# EUROPEAN PATENT APPLICATION

(11) **EP 4 501 273 A1**
(43) Date of publication of application: **05.02.2025**
(21) Application number: 23774753.0
(22) Date of filing: 16.03.2023
(51) Int. Cl.: A61C 5/40

(54) **LIQUID DRUG TRANSPORT DEVICE, LIQUID DRUG TRANSPORT SYSTEM, AND LIQUID DRUG TRANSPORT METHOD**

(30) Priority: 25.03.2022 JP 2022049927
(71) Applicant: Tohoku University, Sendai-shi, Miyagi 980-8577 (JP)
(72) Inventor: YAHATA, Yoshio, Sendai-shi, Miyagi 980-8577 (JP); SAITO, Masahiro, Sendai-shi, Miyagi 980-8577 (JP); SOYAMA, Hitoshi, Sendai-shi, Miyagi 980-8577 (JP)
(74) Representative: Studio Torta S.p.A.
(86) International application number: PCT/JP2023/010364
(87) International publication number: WO 2023/182157

(57) **Abstract**

A liquid drug delivery device 5 for delivering a liquid drug to a target site 2a in a living tissue 2 by irradiating the liquid drug with laser light L at a location 1a near the target site 2a.

## Description

### FIELD

The present invention relates to technologies for delivering a liquid drug to a target site in a living tissue.

### BACKGROUND

When left untreated, dental pulpitis progresses to pulp necrosis and pulp gangrene, leading to bacterial infection spreading into the periapical tissues, resulting in apical periodontitis. When apical periodontitis develops, pus accumulates inside the alveolar bone, causing destruction of the jawbone that supports the tooth. Current dental treatments involve performing an infected root canal treatment to remove bacteria and address apical periodontitis (for example, Non-Patent Document 1).

### Related Art Documents

### Non-Patent Documents

[Non-Patent Document 1] Takatoku Takenawa, "APICAL PERIODONTITIS", Social Welfare Organization Saiseikai Imperial Gift Foundation, Inc. August 2, 2017, Internet <URL:https://www.saiseikai.or.jp/medical/disease/apical_per iodontitis/>

### SUMMARY OF THE INVENTION

### Technical Problem

Patients with underlying conditions such as inflammatory bowel disease (IBD) often experience deteriorating oral environments, leading to treatmentresistant apical periodontitis, thus causing persistent jawbone destruction. For such refractory apical periodontitis, current standard treatments often involve repeated procedures such as irrigating the affected site with disinfectants like sodium hypochlorite solution after removing the dental pulp, or surgically removing accumulated pus. However, the effectiveness of these treatments greatly depends on the body's natural healing abilities, and achieving a complete cure through these standard treatments remains challenging.

Therefore, the inventors of the present invention independently investigated ways to inject liquid drugs such as an anti-inflammatory agent into the site from which the dental pulp has been removed, aiming to suppress inflammation that has spread to a periapical tissues and jawbone. However, due to the extremely narrow space created by the removal of the dental pulp, it was difficult to ensure that the liquid drug reached not only the root canal apex but also the jawbone connected thereto, mainly due to the formation of air bubbles during injection.

Hence, the present invention was devised to address the above-described problem of facilitating the delivery of liquid drugs to the target site in living tissues.

### Solution to Problem

Upon earnest consideration of the above-described problem, the inventors discovered that the liquid drug could be delivered effectively by irradiating the liquid drug with laser light. The present invention is the result of further exploration based on this insight and is described as follows:
Clause 1. A liquid drug delivery device for delivering a liquid drug to a target site in a living tissue by irradiating the liquid drug with laser light at a location near the target site.
Clause 2. The liquid drug delivery device according to clause 1, wherein the liquid drug is selected from a group comprising an anti-inflammatory agent, a disinfectant, an analgesic, a local anesthetic, a molecularly targeted drug, and a physiological saline solution.
Clause 3. The liquid drug delivery device according to clause 1 or 2, wherein the target site is a jawbone.
Clause 4. The liquid drug delivery device according to any one of clauses 1 to 3, wherein the location near the target site is in a root canal from which dental pulp of a tooth adjacent to the jawbone has been removed.
Clause 5. A liquid drug delivery system comprising:
   an injector for injecting a liquid drug into a location near a target site in a living tissue, and a liquid drug delivery device as described in any one of clauses 1 to 4.
Clause 6. The liquid drug delivery system according to clause 5, wherein the injector is a prefilled syringe.
Clause 7. A liquid drug delivery method comprising steps of injecting a liquid drug into a location near a target site in a living tissue and delivering the liquid drug to the target site by irradiating the liquid drug with laser light.

### Effect of the Invention

According to the present invention, a liquid drug can be easily delivered to a target site in a living tissue.

### BRIEF DESCRIPTION OF THE DRAWINGS

Fig. 1 schematically illustrates a step of a liquid drug delivery method according to an embodiment of the present invention;
Fig. 2 schematically illustrates a step of a liquid drug delivery method according to an embodiment of the present invention;
Fig. 3 schematically illustrates a step of a liquid drug delivery method according to an embodiment of the present invention;
Fig. 4 schematically illustrates a liquid drug delivery system according to an embodiment of the present invention;
Fig. 5 is a photograph of a model for storing a liquid drug that is irradiated with laser light;
Fig. 6 is a sequence of photographs while a rectangular frame shown in Fig. 5 is irradiated with laser light;
Fig. 7 is a photograph showing the experimental setup for delivering a liquid drug to the jawbone of a rat;
Fig. 8 is a cross-sectional photograph showing a first molar and jawbone after irradiation with laser light;
Fig. 9(a) shows a cross-sectional CT image of a jaw area (first molar and jawbone) before the liquid drug is delivered there;
Fig. 9(b) shows a 3D reconstructed image of the jaw area before the liquid drug is delivered there;
Fig. 10(a) shows a cross-sectional CT image of the jaw area after the liquid drug is injected there;
Fig. 10(b) shows a 3D reconstructed image of the jaw area after the liquid drug is injected there;
Fig. 11(a) shows a cross-sectional CT image of the jaw area after the injected liquid drug is irradiated with laser light;
Fig. 11(b) shows a 3D reconstructed image of the jaw area after the injected liquid drug is irradiated with laser light;
Fig. 12(a) is an example of a cross-sectional CT image of a jaw area in a control group;
Fig. 12(b) is an image that highlights the jawbone destructed region in the image shown in Fig. 12 (a);
Fig. 13(a) is an example of a cross-sectional CT image of a jaw area in an experimental group using a physiological saline solution;
Fig. 13(b) is an image that highlights the jawbone destructed region in the image shown in Fig. 13 (a);
Fig. 14(a) is an example of a cross-sectional CT image of the jaw area in the experimental group using an anti-inflammatory agent;
Fig. 14(b) is an image that highlights the jawbone destructed region in the image shown in Fig. 14 (a);
Fig. 15 is a graph showing the volume of jawbone destructed regions in the control group after only root canal treatment (RCT), the experimental group after treatment with physiological saline solution, and the experimental group after treatment with an anti-inflammatory agent;
Fig. 16(a) is an exploded perspective view of a liquid drug delivery device;
Fig. 16(b) is a cross-sectional view of the liquid drug delivery device;
Fig. 17 schematically illustrates a method for delivering a liquid drug into a jawbone using the liquid drug delivery device shown in Fig. 16;
Fig. 18(a) shows a cross-sectional CT image before liquid drug delivery;
Fig. 18(b) shows a cross-sectional CT image after liquid drug delivery;
Fig. 19(a) shows a cross-sectional image before drug injection;
Fig. 19(b) shows a cross-sectional image after drug injection but before laser light irradiation;
Fig. 19(c) shows a cross-sectional image after laser light irradiation that is generated under the output conditions of 3 pulses per second, 30 millijoules, and 3 seconds of irradiation time;
Fig. 20(a) shows a cross-sectional image after laser light irradiation that is generated under the conditions of 3 pulses per second, and 80 millijoules;
Fig. 20(b) shows a cross-sectional image after laser light irradiation that is generated under the conditions of 3 pulses per second, and 150 millijoules;
Fig. 20(c) shows a cross-sectional image after laser light irradiation that is generated under the conditions of 3 pulses per second, and 350 millijoules;
Fig. 21(a) illustrates the steps for preparing rats with a destructed jawbone;
Fig. 21(b) shows cross-sectional images of a non-treatment group, a standard treatment group, and a DDS group;
Fig. 21(c) is a graph showing the area of destructed regions for the non-treatment group, the standard treatment group, and the DDS group;
Fig. 22 (a) illustrates the steps for preparing rats with systemic diseases complicating jawbone destruction;
Fig. 22(b) shows cross-sectional images of a non-treatment group, a standard treatment group, and a DDS group; and
Fig. 22(c) is a graph showing the area of destructed regions for the non-treatment group, the standard treatment group, and the DDS group.

### Description of Embodiments

Hereinafter, an embodiment according to the present invention is described with reference to the accompanying drawings. However, the present invention is not limited to the embodiments described below and various changes can be made without departing from the spirit thereof.

### (Summary)

The liquid drug delivery device according to the present invention is configured to deliver a liquid drug to a target site by irradiating the liquid drug with laser light at a location near the target site in a living tissue. For example, the liquid drug is selected from a group comprising an anti-inflammatory agent, a disinfectant, an analgesic, a local anesthetic, a molecularly targeted drug, and a physiological saline solution. The target site is a jawbone partially destructed due to apical periodontitis. The location near the target site is in the root canal from which dental pulp of a tooth adjacent to the jawbone has been removed.

Further, the liquid drug according to the present invention is delivered to the target site in a living tissue, the liquid drug being delivered to the target site by irradiating the liquid drug with laser light at a location near the target site.

Further, the liquid drug delivery method according to the present invention comprises an injection step for injecting a liquid drug into a location near a target site in a living tissue, and a delivery step for delivering the liquid drug to the target site by irradiating the liquid drug with laser light.

### (Steps for delivery)

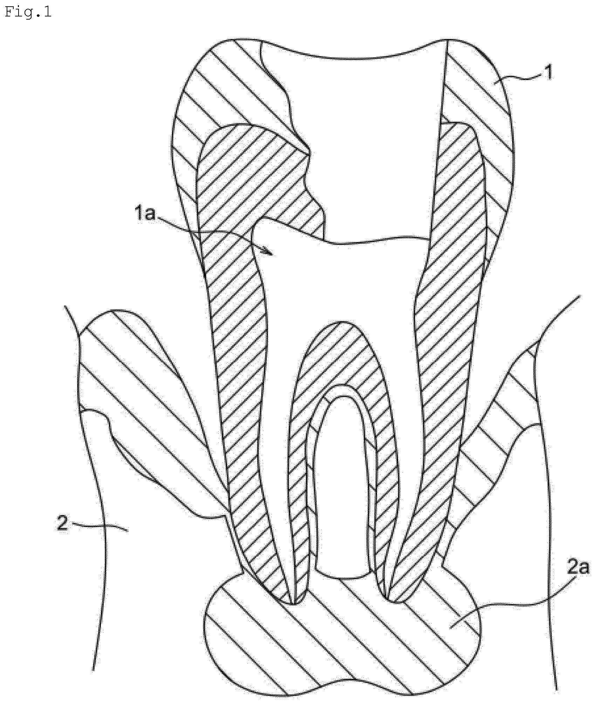

In the present embodiment, a bone is described as an example of the living tissue. Figs. 1 to 3 schematically illustrate the steps for the liquid drug delivery method according to the present embodiment.

As shown in Fig. 1, the jawbone 2 that supports tooth 1 has experienced jawbone destruction around the tooth root due to the invasion of bacteria causing apical periodontitis. In this embodiment, the area around the tooth root is designated as a target site 2a, and a liquid drug such as an anti-inflammatory agent is delivered to the target site 2a. In the preparation stage of the delivery, a hole is formed in the tooth 1 next to the jawbone 2, and the dental pulp is removed therethrough. A root canal 1a refers to the part from which the dental pulp has been removed.

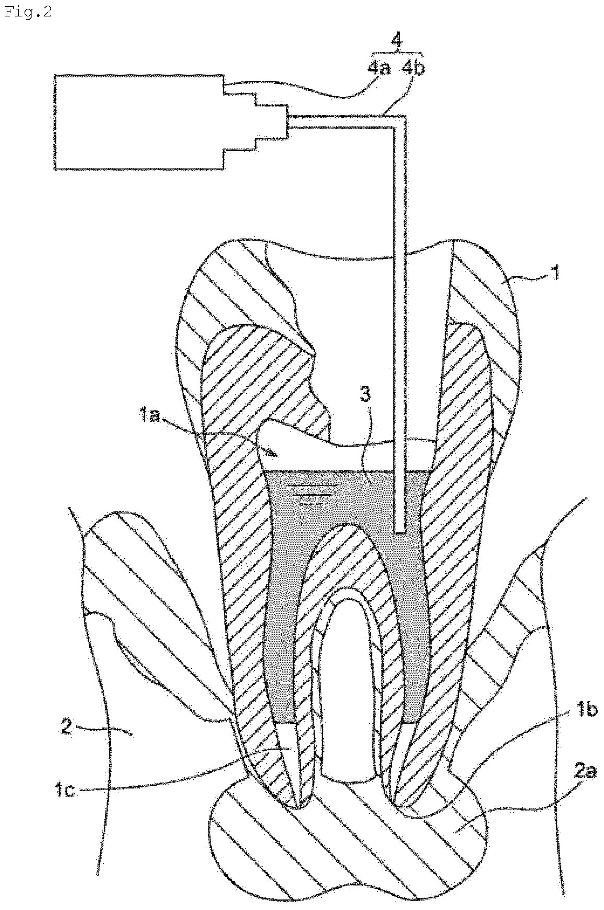

Next, an injection process is performed. In this embodiment, as shown in Fig. 2, a liquid drug 3 is injected into the root canal 1a using an injector 4, thus delivering the liquid drug 3 to a location near the target site 2a. The injector 4 comprises a syringe 4a for containing the liquid drug 3 and a needle 4b to be inserted into the root canal 1a.

As shown in Fig. 2, the apical foramen 1b leading to the jawbone 2 is extremely small, preventing the liquid drug 3 from reaching the tip of the root canal 1a. Consequently, the liquid drug 3 does not permeate the jawbone 2. Additionally, in many cases, air bubbles 1c are mixed with the liquid drug 3, blocking the area near the tip of the root canal 1a due to these air bubbles 1c. Because these air bubbles 1c are extremely tiny, removing them using the needle 4b is difficult.

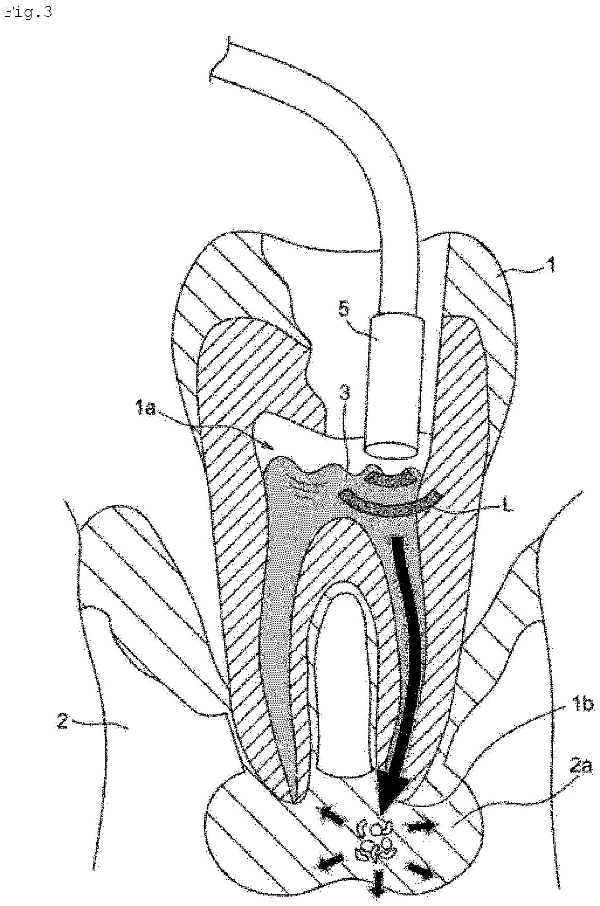

Next, the delivery process is performed. In this embodiment, as shown in Fig. 3, the liquid drug 3 is irradiated with laser light L using a laser irradiation device 5, delivering the liquid drug 3 to the tip of the root canal 1a, and further delivering the same to the target site 2a. Specifically, by irradiating the liquid drug 3 with laser light L of appropriate intensity and pulse frequency, air bubbles are generated near the irradiation site of the liquid drug 3 (shallow part) due to laser-induced stress waves (laser ablation). In the deeper part of the liquid drug 3, microbubbles (laser cavitation) occur due to the pressure difference between the shallow part and the deeper part. Consequently, an external mechanical force is applied to the liquid drug 3, delivering the liquid drug 3 to the tip of the root canal 1a and pushing the same out into the jawbone 2 through the apical foramen 1b, thus delivering the liquid drug 3 to the target site 2a.

The intensity, pulse frequency, and power (intensity × pulse frequency) of the laser light L are not particularly limited, as long as the liquid drug 3 can be delivered to the target site 2a. For example, it is preferable for the intensity to be in the range of 10 to 30 millijoules. The pulse frequency is preferably 1 to 10 pulses per second, more preferably 3 to 10 pulses per second. The power is preferably 10 to 1000 milliwatts, more preferably 90 to 200 milliwatts. Note that if the intensity and/or pulse frequency of the laser light L are too low, sufficient external mechanical force may not be applied to the liquid drug 3, preventing the liquid drug 3 from being delivered to the target site 2a. On the other hand, if the intensity and/or pulse frequency of the laser light L are too high, the liquid drug 3 may evaporate, or damage to the jawbone may occur. For reference, in dental treatments using laser light to cut a tooth, the power is approximately 2000 to 3500 milliwatts.

The tip position of the laser irradiation device 5 is preferably adjusted to generate laser cavitations near the apical foramen 1b. In Fig. 3, the laser irradiation device 5 is positioned apart from the surface of the liquid drug 3, however, the laser irradiation device 5 can also be immersed in the liquid drug 3. Further, the laser irradiation device 5 corresponds to the liquid drug delivery device described in claims.

In this embodiment, as described above, the liquid drug 3 is injected into the location near the target site 2a in the jawbone 2. Subsequently, the liquid drug 3 can be easily delivered to the target site 2a in the jawbone 2 by irradiating the liquid drug 3 with laser light L.

The above description pertains to an embodiment involving the delivery of a liquid drug to the jawbone via a root canal in dental treatment. However, the present invention is not limited to this embodiment and can also be applied to the delivery of a liquid drug to diseases other than apical periodontitis and bones other than the jawbone. While not restricting the application of the present invention, the present invention can be applied to deliver a liquid drug to the affected area in a bone, for example, in the treatment of hard tissue diseases such as chronic rheumatoid arthritis, chronic recurrent multifocal osteomyelitis, pyogenic osteomyelitis, and the like. In this case, if there is a liquid-drug injectable part at the location near the affected area in the bone, the liquid drug is injected into that part, but if there is no such injectable part, the liquid drug is conveyed to the location near the target site in the living tissue using a reservoir such as a prefilled syringe to be inserted into the living tissue, and then the liquid drug at the location near the target site can be delivered to the target site by irradiating the liquid drug with laser light. Additionally, even if there is a liquid-drug injectable part at the location near the affected area in the bone, the liquid drug can also be conveyed to the location near the target site using the reservoir.

The location near the target site in a living tissue refers to a position within the range in which the liquid drug 3 can be delivered from said position to the target site by irradiating the liquid drug 3 with laser light L. In the above embodiment, the location near the target site 2a in the jawbone 2 refers to the liquid drug storage part inside the root canal 1a shown in Fig. 2. In the case of chronic rheumatoid arthritis, the location near the target site in a living tissue refers to the position near the affected area inside the bone (the target site in the living tissue) that needs to be treated in chronic rheumatoid arthritis. For example, the location near the target site refers to a position from which the liquid drug can be delivered to the target site by irradiating the liquid drug retained in the above-described part capable of storing a liquid drug or a reservoir (hereinafter, both may be referred to as a storage part). Although not limiting the present invention, it is preferable, for instance, that the distance (straight-line distance) between the target site and the liquid drug stored in the storage part is 5 mm or less, and more preferably 1 to 3 mm. According to Fig. 2, the distance between the bottom surface of the liquid drug 3 stored in the root canal 1a (bottom surface of the liquid drug storage part) and the apical foramen 1b is approximately 2 mm.

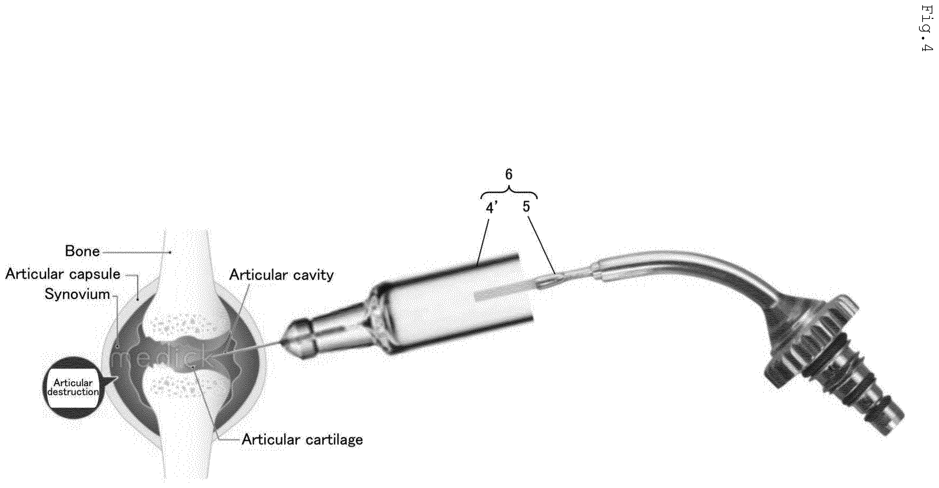

When using a reservoir, the shape of the reservoir is not limited, but a form utilizing a prefilled syringe is shown in Fig. 4. Fig. 4 is a schematic diagram illustrating the Liquid Drug Delivery System (DDS) 6 according to the present embodiment. The liquid drug delivery system 6 comprises a prefilled syringe 4', which serves as an injector, and a laser irradiation device 5. The prefilled syringe 4' is filled with a liquid drug such as an anti-inflammatory agent.

As shown in Fig. 4, an injection needle connected to the prefilled syringe 4' is inserted into the affected area in the bone (articular cavity) and brought into the location near the target bone tissue. During this process, it is desirable for the tip of the injection needle to be positioned within 5 mm from the target bone tissue and even more desirable for the tip of the injection needle to be in direct contact with the target bone tissue. A film (e.g. polyethylene film) is attached at the other end of the prefilled syringe 4', and the tip of the laser irradiation device 5 is inserted into the syringe through the film. The liquid drug can be delivered to the target bone tissue via the injection needle by irradiating the inside of the prefilled syringe 4' with laser light using the laser irradiation device 5.

The injected liquid drug can be appropriately determined based on the disease or the target site in the bone. Any injected liquid drug can be used as long as the liquid drug remains in the form of liquid at room temperature (25°C). The injected liquid drug can also be formed by mixing solid drugs (including freeze-dried drugs, etc.) with liquids such as physiological saline solution or injectable water.

In this way, the liquid drug can be easily delivered to the target site by injecting the liquid drug into the location near the target site in a living tissue and subsequently irradiating the liquid drug with laser light.

### Examples

Hereinafter, examples of the present invention are described below, but the present invention is not limited to these examples.

### (Example 1)

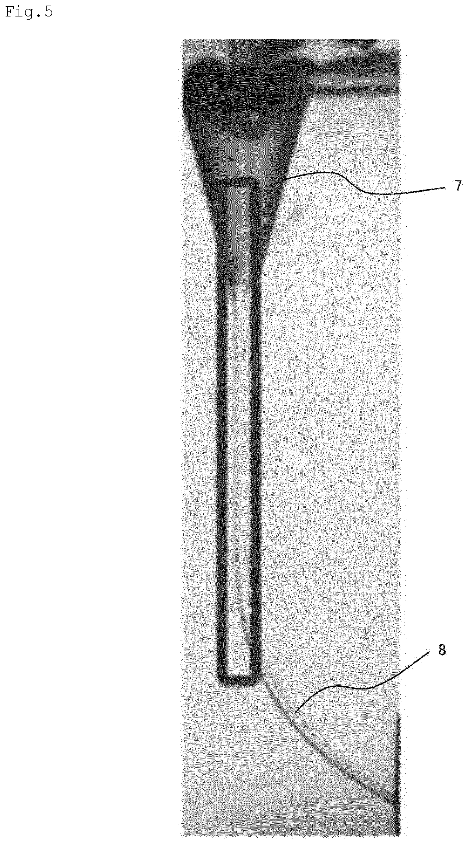

In example 1, a transparent acrylic component was used to create a cavity resembling a tooth's root canal and deaerated distilled water was injected into the cavity. The cavity consists of a funnel-shaped storage part 7 and a tube part 8 that extends from the lower end of the storage part 7, as shown in Fig. 5. The tube part 8 is 16 mm long with a diameter of 0.15 mm at the bottom and a 2/100 taper. Subsequently, the liquid drug was irradiated with laser light. The laser light had an intensity of 30 millijoules and a pulse frequency of 3 pulses per second.

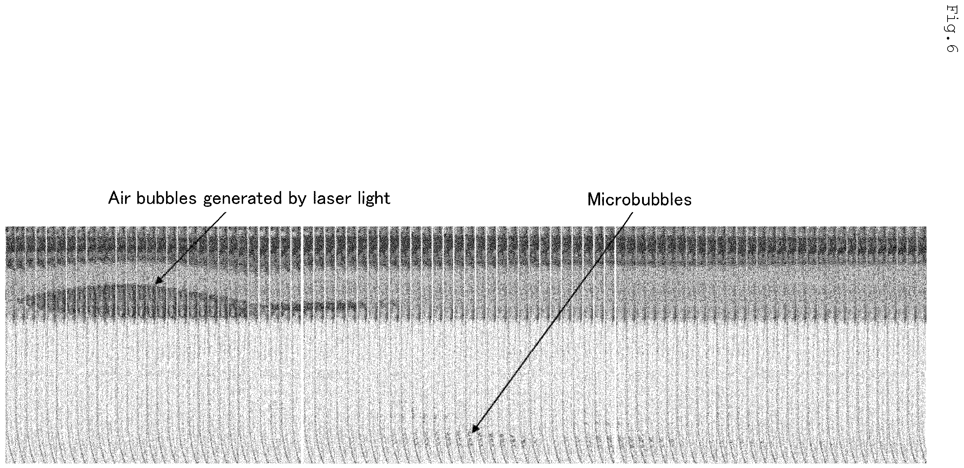

Fig. 6 is a series of consecutive photographs taken during laser light irradiation within the rectangular frame shown in Fig. 5. These photos were captured with a shutter speed of 1/72,000 seconds and are arranged chronologically from left to right. Upon irradiation with laser light, air bubbles first appeared in the shallow part of the liquid drug, followed by the generation of microbubbles in the deeper part of the liquid drug (approximately 8 mm from the bottom of the storage part 7). With these microbubbles, it was confirmed that the liquid drug can be intentionally delivered to the very tip of the extremely fine tube part 8 by irradiating the liquid drug with laser light.

Therefore, it was learned that the liquid drug can be delivered into the jawbone in dental treatments by irradiating the liquid drug stored in the root canal with laser light to generate microbubbles near the boundary between the tooth and the jawbone (apical foramen).

### (Example 2)

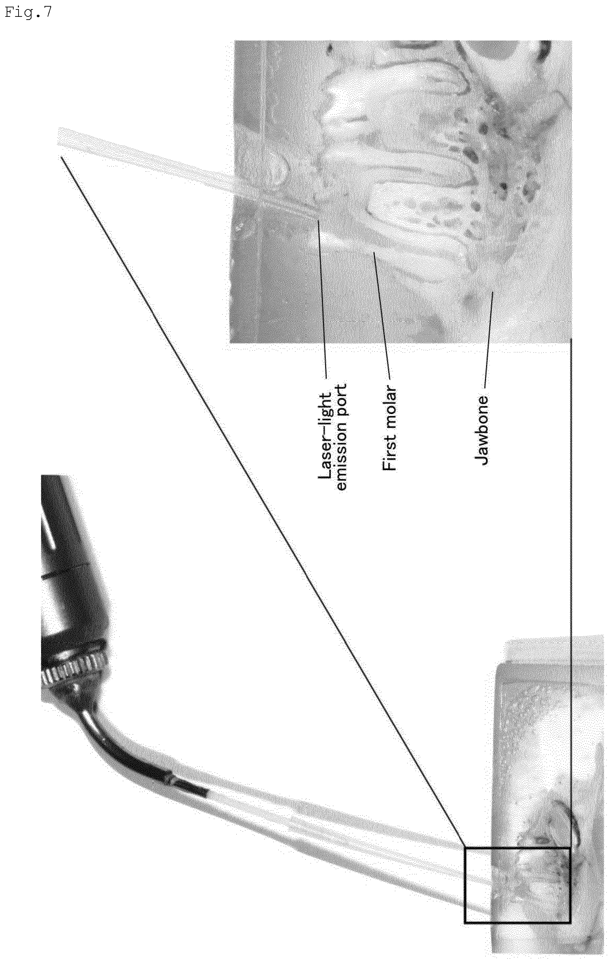

In example 2, an experiment was conducted to deliver a liquid drug into the jawbone of a rat. Specifically, as shown in Fig. 7, a liquid drug was injected into the interior of the rat's first molar (root canal). Subsequently, the tip of the laser irradiation device (laser light emission port) was positioned inside the tooth (shallow part), and thus irradiates the inside of the tooth with laser light. The intensity of the laser light was 30 millijoules, and the pulse frequency was 3 pulses per second.

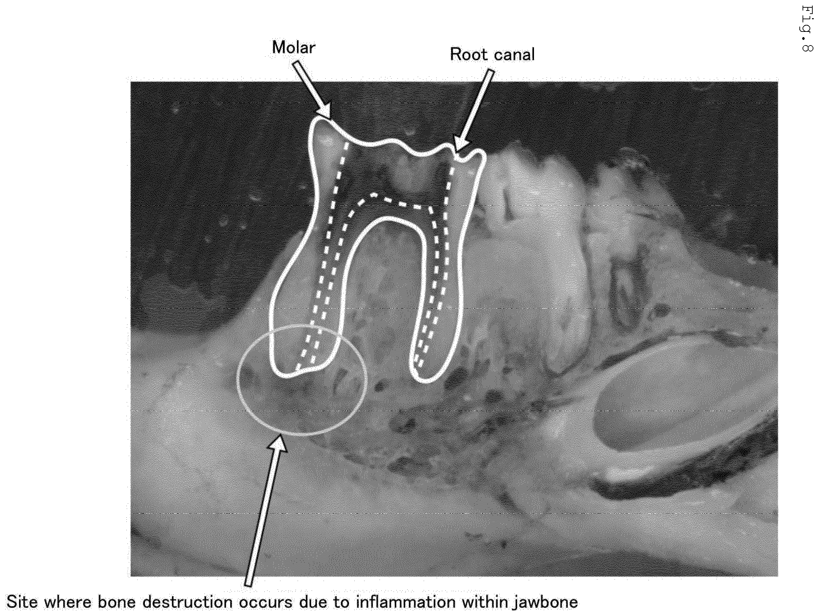

Fig. 8 is a cross-sectional photograph of a first molar and a jawbone after being irradiated with laser light. It can be seen that the delivered liquid drug has permeated the jawbone.

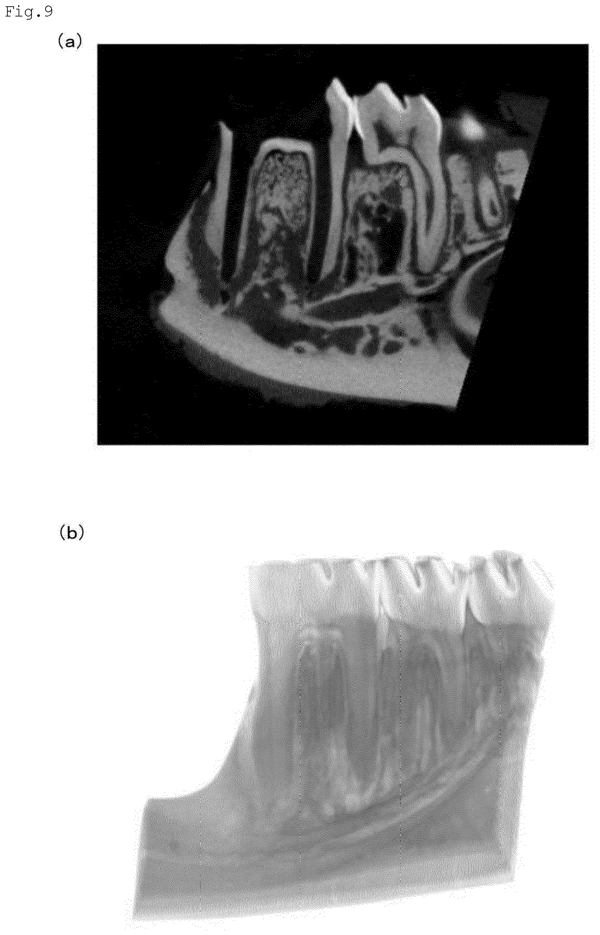

Figs. 9(a) and 9(b) respectively show a cross-sectional CT image and a 3D reconstructed image of a jaw area (first molar and jawbone) before the liquid drug is injected there.

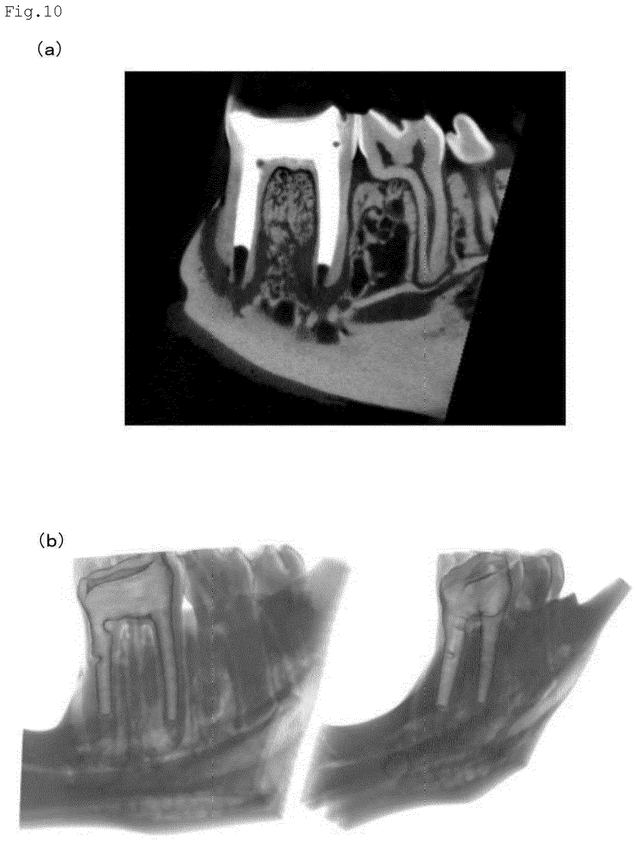

Figs. 10(a) and 10(b) respectively show a cross-sectional CT image and a 3D reconstructed image of the jaw area after the liquid drug is injected there. The liquid drug is shown white in the cross-sectional CT image and shown blue in the 3D reconstructed image. As air bubbles exist at the tip of the root canal, it can be seen that the liquid drug is retained at the lower part of the root canal.

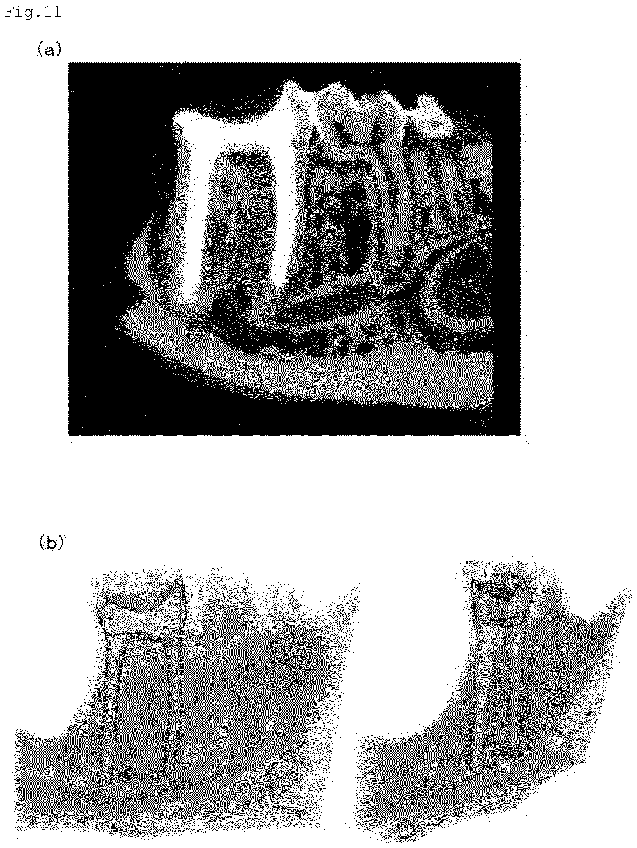

Figs. 11(a) and 11(b) respectively show a cross-sectional CT image and a 3D reconstructed image of the jaw area after the injected liquid drug is irradiated with laser light. It can be seen that the liquid drug has permeated the jawbone from the lower part of the root canal.

### (Example 3)

In example 3, rats with jawbone inflammation were targeted to deliver a liquid drug to the site of jawbone destruction, thereby verifying the therapeutic effect. Specifically, the liquid drug was injected into the root canal, and then laser light was applied to the liquid drug. The experimental group, which received the delivery of the liquid drug to the site of jawbone destruction through laser irradiation, was compared with the control group, which underwent only the process of liquid drug injection into the root canal, in terms of the volume of jawbone destructed regions after treatment. The intensity of the laser light was 30 millijoules, and the pulse frequency was 3 pulses per second. Physiological saline solution or an anti-inflammatory drug (triamcinolone) was used as the liquid drug. Cross-sectional CT images of the jaw area were taken after 28 days of treatment.

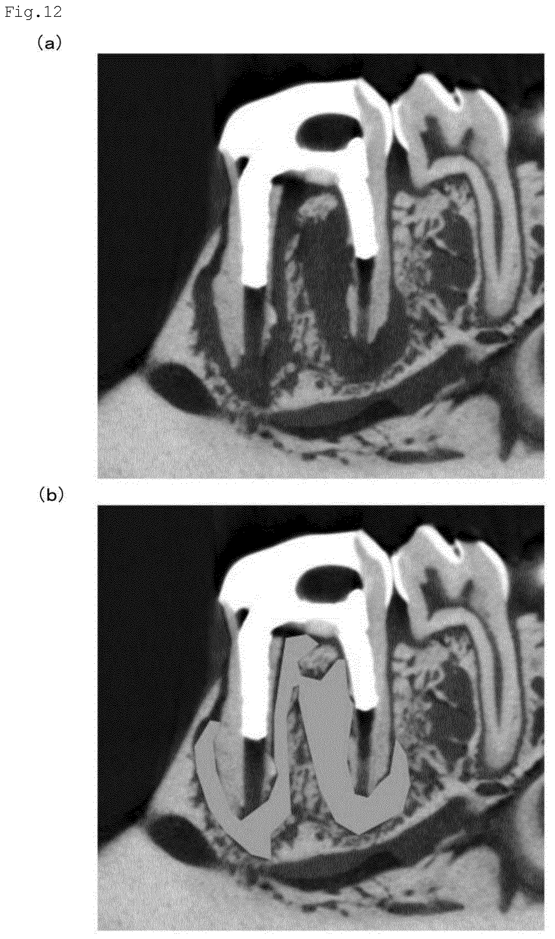

Fig. 12(a) shows an example of a cross-sectional CT image of the jaw area from the control group, and Fig. 12(b) shows an image emphasizing the destructed part of the jawbone in the image shown in Fig. 12(a). The volume of the jawbone destructed region remained unchanged compared to before the treatment.

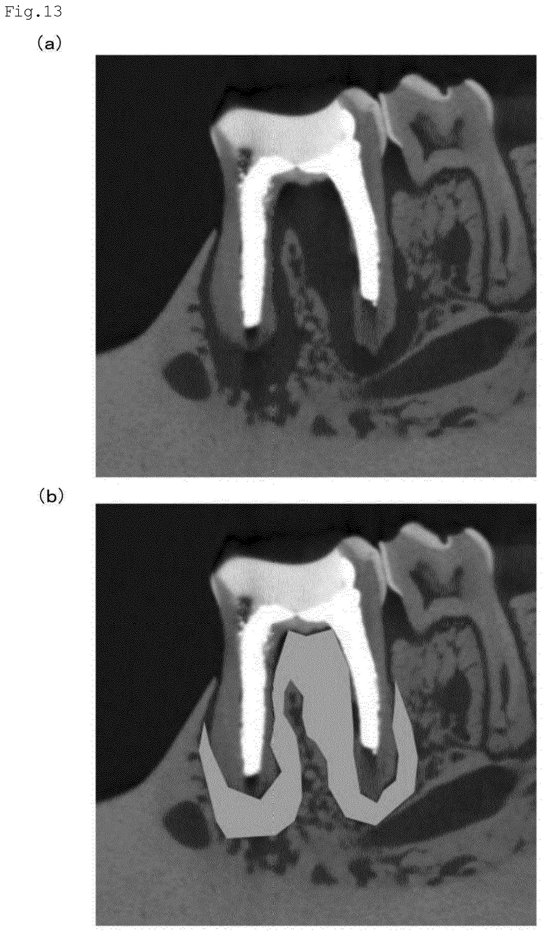

Fig. 13(a) shows a cross-sectional CT image of the jaw area from the experimental group treated with saline solution, and Fig. 13(b) shows an image emphasizing the destructed part of the jawbone in the image shown in Fig. 13(a). The volume of the jawbone destructed region had slightly decreased compared to before the treatment.

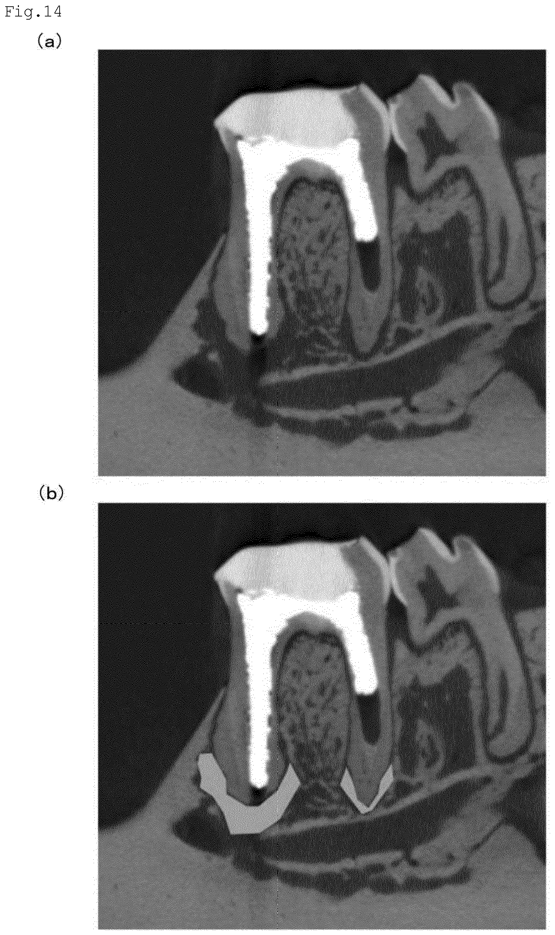

Fig. 14(a) shows a cross-sectional CT image of the jaw area from the experimental group treated with an anti-inflammatory drug, and Fig. 14(b) shows an image emphasizing the destructed part of the jawbone in the image shown in Fig. 14(a). The volume of the jawbone destructed region had significantly decreased compared to before the treatment.

It should be noted that in Figs. 12 to 14, the white areas indicated in the canal roots do not show liquid drugs.

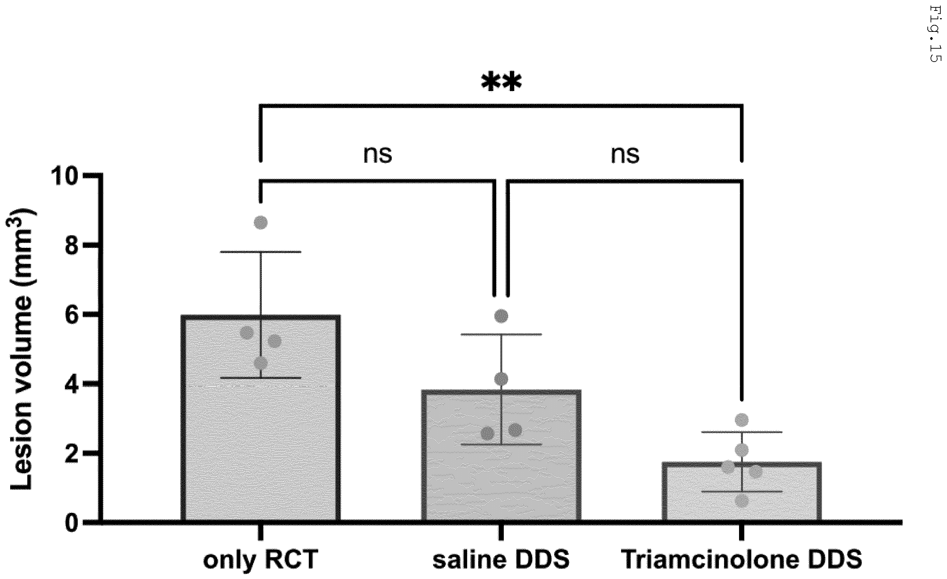

Fig. 15 is a graph illustrating the volume of jawbone destructed regions after treatment for the control group shown in Fig. 12 (only Root Canal Treatment (RCT)), the experimental group treated with saline solution shown in Fig. 13 (Saline DDS), and the experimental group treated with an anti-inflammatory drug shown in Fig. 14 (Triamcinolone DDS). From this graph, it can be seen that the jawbone destructed region has significantly reduced by delivering the liquid drug to the jawbone destructed region through laser light irradiation.

### (Example 4)

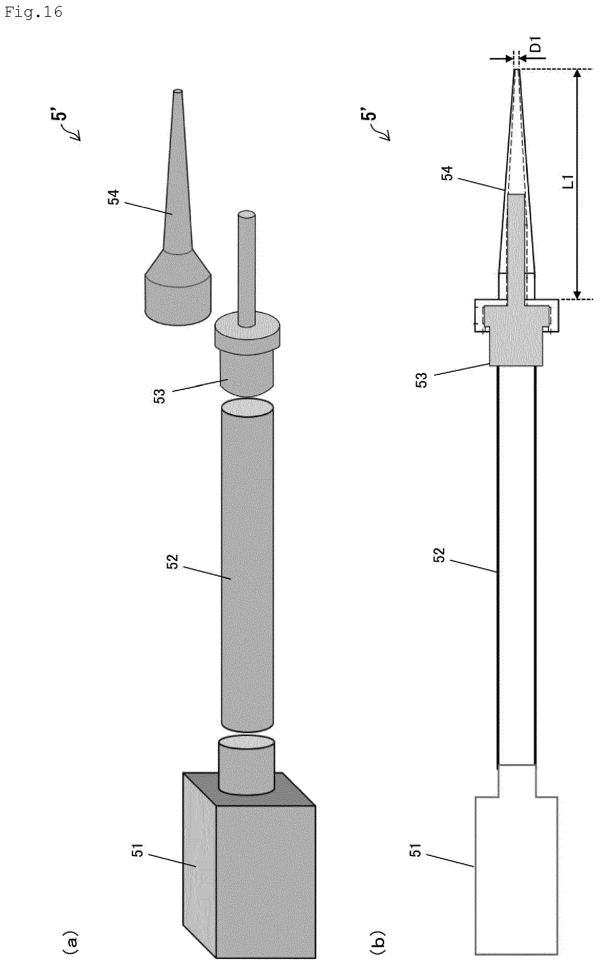

Example 4 involved conducting experiments for delivery of a liquid drug using a liquid drug delivery device 5' shown in Fig. 16. Figs. 16 (a) and (b) represent an exploded perspective view and a cross-sectional view of the liquid drug delivery device 5', respectively. The liquid drug delivery device 5' comprises a pulse laser oscillator 51, a laser transmission cable 52, a contact chip 53, and a prefilled syringe 54.

The pulse laser oscillator 51 is linked to one end of the laser transmission cable 52 and is designed to emit a pulse laser with a wavelength of 2.94 um into the laser transmission cable 52. The contact chip 53 serves as a component connecting the laser transmission cable 52 and the prefilled syringe 54. The total length of the laser transmission cable 52 and the contact chip 53 is approximately 1 meter. The prefilled syringe 54 has a tapered protrusion. The length L1 of the protrusion measures 21 mm, and the diameter D1 at the tip of the protrusion is 0.3 mm. The protrusion is hollow with liquids stored therein.

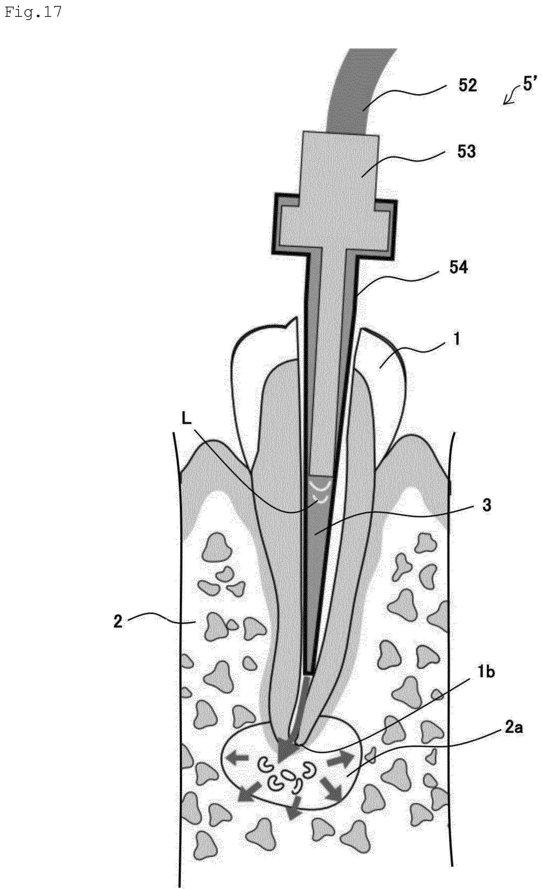

Fig. 17 is a schematic diagram illustrating the method of delivering a liquid drug into the jawbone using the liquid drug delivery device 5'. Inside the protrusion of the prefilled syringe 5', the liquid drug 3 is stored. The protrusion of the prefilled syringe 5' is inserted into the root canal of the tooth 1, and the tip of the protrusion is positioned approximately 5 mm away from the apical foramen 1b. At this point, the tip of the contact chip 53 is about 10 mm away from the apical foramen 1b. In this state, the liquid drug 3 is irradiated with laser light L emitted from the tip of the contact chip 53. As a result, mechanical force is applied to the liquid drug 3, causing the same to be forcefully ejected from the tip of the protrusion and delivered from the apical foramen 1b to the target site 2a in the jawbone 2.

In the experiment, a liquid was injected into the inside of a dental model for observation resembling a human tooth using the liquid drug delivery device 5'. The laser output was 30 millijoules. As a result, it was confirmed that the liquid could be delivered into the jawbone of the dental model for observation.

Furthermore, when the sound pressure in the jawbone (corresponding to the apical lesion) was measured using a hydrophone during laser light irradiation (30 millijoules, 80 millijoules, 100 millijoules), sound pressure changes were observed at all output levels.

Additionally, the liquid drug delivery device 5' was used to examine the feasibility of delivering liquid drugs into the inside of a human jawbone. Specifically, a drug substance (contrast agent) was loaded into the inside of the protrusion of the prefilled syringe 5', and the protrusion was inserted into the root canal. Laser light L was then applied to the drug substance under the output conditions of 3 pulses per second, 30 millijoules.

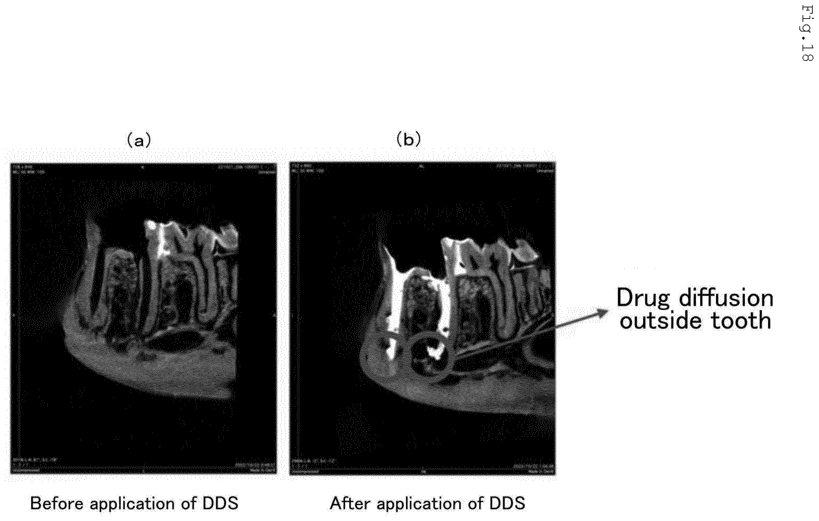

Fig. 18 (a) shows the CT cross-sectional image before a liquid drug delivery, and Fig. 18 (b) shows the CT cross-sectional image after the liquid drug delivery. It can be confirmed from Fig. 18 (b) that the drug substance has diffused into the jawbone.

### (Example 5)

In Example 5, optimal output conditions for laser light were examined. Specifically, in the same way as Example 2, a liquid drug was injected into the inside of the first molar (root canal) in a rat. Subsequently, the tip of the laser irradiation device (laser light emission port) was placed inside the tooth (shallow part), and laser light irradiation was performed under different output conditions. During the laser light irradiation, the jawbone of the rat was continuously captured to confirm the diffusion of the liquid drug inside the jawbone.

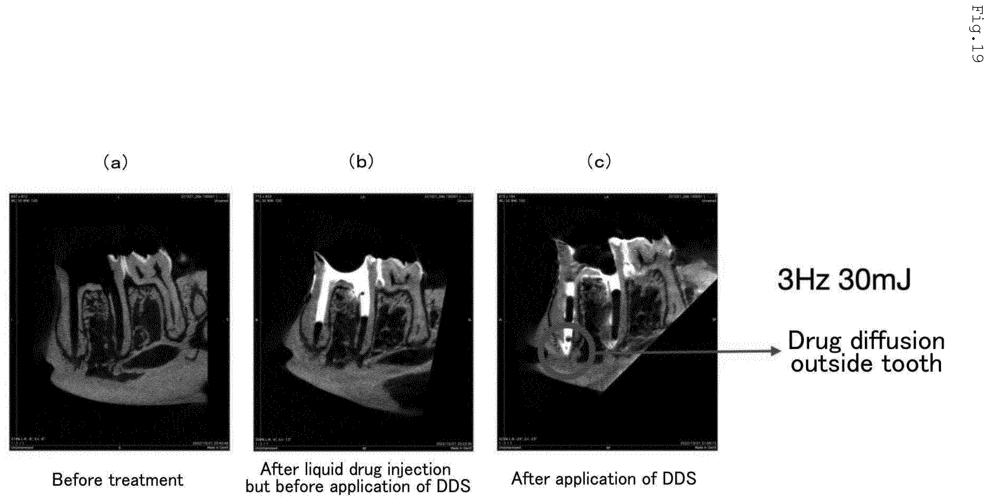

Fig. 19(a) shows a cross-sectional image before drug injection, Fig. 19(b) shows a cross-sectional image after drug injection but before laser light irradiation, and Fig. 19(c) shows a cross-sectional image after laser light irradiation under the output conditions of 3 pulses per second, 30 millijoules, and an irradiation time of 3 seconds. In this case, it was confirmed that the drug had diffused outside the tooth. However, even under the same conditions, when laser light at 1 pulse per second, 30 millijoules was applied, drug diffusion outside the tooth was not observed.

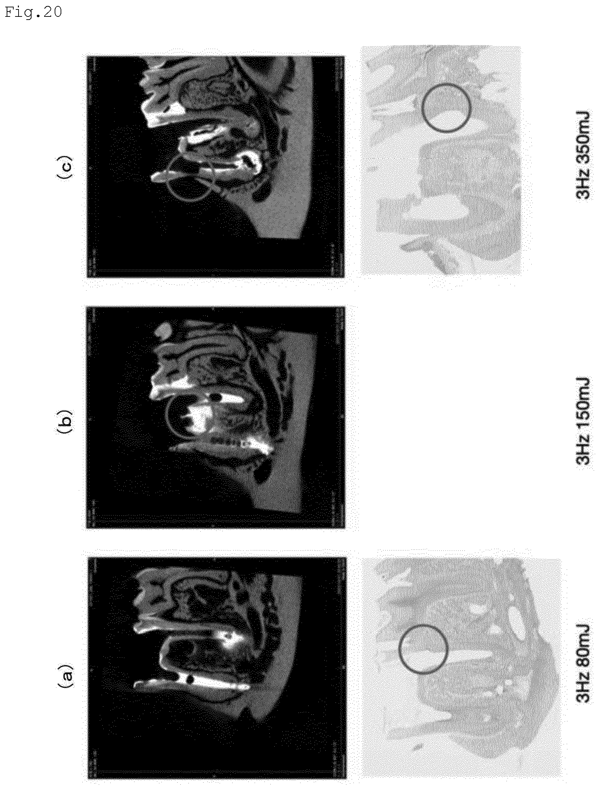

Further experiments were conducted by changing the output conditions. Fig. 20(a) shows a cross-sectional image after laser light irradiation under the output conditions of 3 pulses per second, 80 millijoules, Fig. 20(b) shows a cross-sectional image after laser light irradiation under the output conditions of 3 pulses per second, 150 millijoules, and Fig. 20(c) shows a cross-sectional image after laser light irradiation under the output conditions of 3 pulses per second, 350 millijoules. The irradiation time was 3 seconds for each condition. As can be seen, increasing the output of the laser light did not cause significant change in the diffusion amount. Additionally, the circular marks represent damaged areas, indicating damage to the jawbone due to laser light irradiation.

### (Example 6)

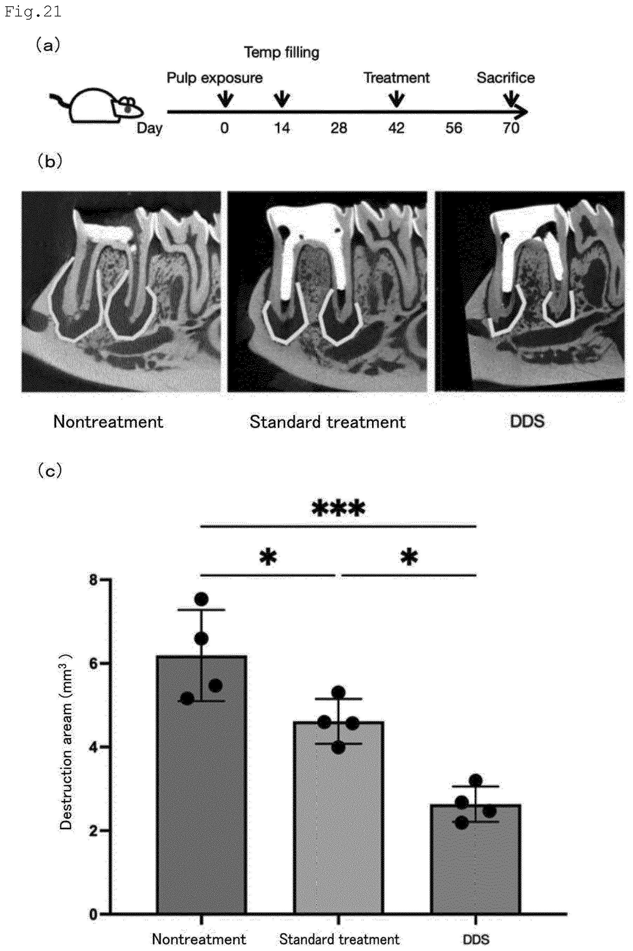

In Example 6, experiments were conducted to verify the therapeutic effects of the liquid drug delivery method according to the present invention on rats with jawbone destruction. Specifically, twelve rats were infected with bacteria (Day 0) and the bacteria were sealed inside the tooth (Day 14) to prepare rats with jawbone destruction, as shown in Fig. 21(a). Subsequently, four rats received no treatment (untreated group), four rats received standard treatment on Day 42 (standard treatment group), and four rats underwent treatment on Day 42 using the liquid drug delivery method according to the present invention to deliver an anti-inflammatory drug into the jawbone (DDS group). After then, on Day 70, cross-sectional images of the rats' jawbones were taken.

Fig. 21(b) shows cross-sectional images of the untreated group, standard treatment group, and DDS group, and Fig. 21(c) shows a graph indicating the area of the destructed regions in the untreated group, standard treatment group, and DDS group. These results confirm that delivery of the drug through the liquid drug delivery method according to the present invention leads to a greater suppression of jawbone destruction and a greater promotion of healing or regeneration compared to standard treatment.

### (Example 7)

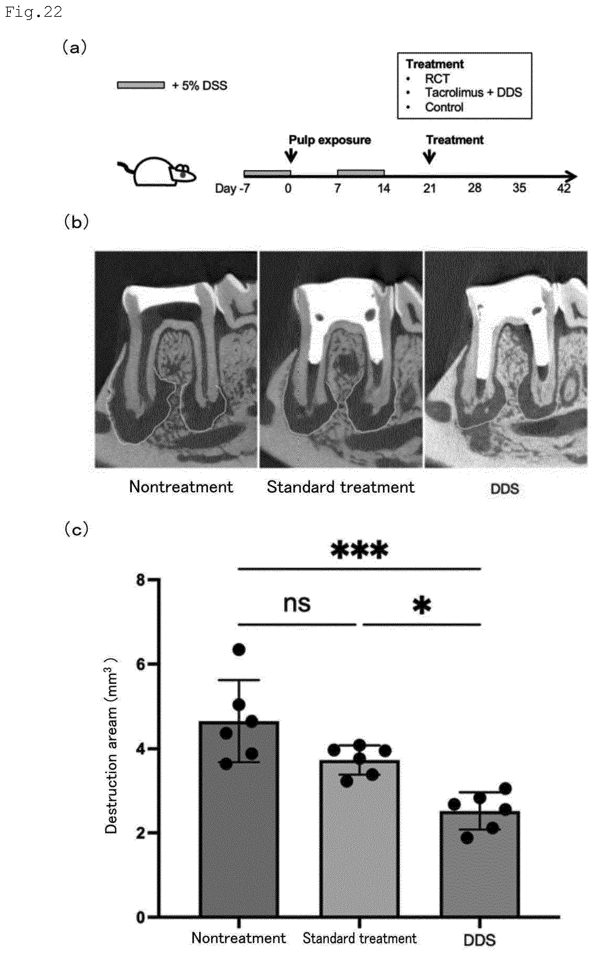

In Example 7, experiments were conducted to examine the therapeutic effects of the liquid drug delivery method according to the present invention on rats with systemic diseases complicating jawbone destruction, which has become a recent issue known as treatment resistance. Specifically, as shown in Fig. 22(a), experiments were conducted on 18 rats by administering dextran sodium sulfate (DSS) (day -7 to day 0, day 7 to day 14) and inducing bacterial infection (day 0), preparing rats with systemic diseases leading to jawbone destruction and coliform infection. Subsequently, 6 rats received no treatment (untreated group), 6 rats underwent standard treatment on day 21 (standard treatment group), and 6 rats were treated by delivering anti-inflammatory drugs to the jawbone using the liquid drug delivery method according to the present invention on day 21 (DDS group). After then, on day 42, cross-sectional images of the rats' jawbones were taken.

Fig. 22(b) shows cross-sectional images of the untreated group, standard treatment group, and DDS group, while Fig. 22(c) shows a graph illustrating the area of the destructed regions in these groups. These results confirm that the treatment using the liquid drug delivery method according to the present invention is more effective than standard treatment even for rats with systemic diseases complicating jawbone destruction.

### Industrial Applicability

The present invention is applicable not only to the delivery of liquid drugs to the jawbone in dental treatments but also to the delivery of liquid drugs to bones other than the jawbone. For instance, in the treatment of hard tissue diseases such as chronic rheumatoid arthritis and chronic recurrent multifocal osteomyelitis, the present invention can be applied to deliver liquid drugs to the affected areas within the bone. In this case, as mentioned above, it is permissible to inject the liquid drug into the location near the target site within the bone using a reservoir such as a prefilled syringe, as needed. The liquid drug can then be delivered to the target site by irradiating the liquid drug with laser light.

Furthermore, the present invention can be applied to the delivery of liquid drugs to any biological tissue other than bones. The target site is not limited to sites affected by diseases and can also include cases where the invention is applied to deliver liquid drugs to healthy tissues, for example, for cosmetic purposes.

### Description of Reference Numerals

1 Tooth
1a Root canal (Location near target site)
1b Apical foramen
2 Jawbone (living tissue)
2a Target site
3 Liquid drug
4 Injector
4a Syringe
4b Needle
4' Prefilled syringe
5 Laser irradiation device (liquid drug delivery device)
5' Liquid drug delivery device
51 Pulse laser oscillator
52 Laser transmission cable
53 Contact chip
54 Prefilled syringe
6 Liquid drug delivery system
7 Storage part
8 Tube part
L Laser light

## Claims

1. A liquid drug delivery device for delivering a liquid drug to a target site in a living tissue by irradiating said liquid drug with laser light at a location near said target site.

2. The liquid drug delivery device according to claim 1, wherein said liquid drug is selected from a group comprising an anti-inflammatory agent, a disinfectant, an analgesic, a local anesthetic, a molecularly targeted drug, and a physiological saline solution.

3. The liquid drug delivery device according to claim 1 or 2, wherein said target site is a jawbone.

4. The liquid drug delivery device according to any one of claims 1 to 3, wherein said location near the target site is in a root canal from which dental pulp of a tooth adjacent to the jawbone has been removed.

5. A liquid drug delivery system comprising:
an injector for injecting a liquid drug into a location near a target site in a living tissue; and
the liquid drug delivery device according to any one of claims 1 to 4.

6. The liquid drug delivery system according to claim 5, wherein said injector is a prefilled syringe.

7. A liquid drug delivery method comprising steps of:
injecting a liquid drug into a location near a target site in a living tissue; and
delivering said liquid drug to said target site by irradiating said liquid drug with laser light.
